# EUROPÄISCHE PATENTSCHRIFT

(11) **EP 3 749 495 B1**
(45) Veröffentlichungstag und Bekanntmachung des Hinweises auf die Patenterteilung: **24.11.2021**
(21) Anmeldenummer: 19716057.5
(22) Anmeldetag: 07.02.2019
(51) Int. Cl.: B27G 1/00

(54) **AUSBESSERUNGSVORRICHTUNG, SOWIE VERFAHREN ZUM AUSBESSERN EINER FEHLSTELLE IN EINEM HOLZWERKSTÜCK**
REPAIR DEVICE, AND METHOD FOR REPAIRING A DEFECT IN A WOODEN WORKPIECE
DISPOSITIF DE RÉPARATION ET PROCÉDÉ DE RÉPARATION D'UN DÉFAUT DANS UNE PIÈCE EN BOIS

(30) Priorität: 09.02.2018 AT 501262018
(43) Veröffentlichungstag der Anmeldung: 16.12.2020
(73) Patentinhaber: Fill Gesellschaft m.b.H., 4942 Gurten (AT)
(72) Erfinder: METZ, Karl, 4970 Eitzing (AT)
(74) Vertreter: Burger, Hannes
(86) Internationale Anmeldenummer: PCT/AT2019/060047
(87) Internationale Veröffentlichungsnummer: WO 2019/153027

(56) Entgegenhaltungen:
- DE-U1- 8 525 512

## Beschreibung

Die Erfindung betrifft eine Ausbesserungsvorrichtung zum Ausbesseren einer Fehlstelle in einem Holzwerkstück, gemäß dem Oberbegriff des Anspruchs 1 sowie ein Verfahren zum Ausbesseren einer Fehlstelle in einem Holzwerkstück gemäß dem Oberbegriff des Anspruchs 10. Eine solche Vorrichtung und ein solches Verfahren sind aus dem Dokument DE 85 25 512 U1 bekannt.

Die AT397368B offenbart eine Vorrichtung zum Ausflicken von Fehlstellen in Holzbrettern mit einer im Wesentlichen längsverlaufenden Faserrichtung und mit einem Magazingehäuse für kreisförmig ausgebildete Holzplättchen, die eine Faserrichtung im Wesentlichen parallel zu ihren Stirnflächen besitzen. Weiters kann vorgesehen sein, dass die Plättchen Farbmarkierungen aufweisen, die zu deren Orientierung beim Einsetzen in die Fehlstellen dienen.

Die EP1792696A1 offenbart eine Vorrichtung zum Ausflicken von Fehlstellen in Holzbrettern mit einer im Wesentlichen längsverlaufenden Faserrichtung und mit einem Magazingehäuse für kreisförmig ausgebildete Holzplättchen, die eine Faserrichtung im Wesentlichen parallel zu ihren Stirnflächen besitzen. Weiters kann vorgesehen sein, dass die Plättchen Farbmarkierungen aufweisen, die zu deren Orientierung beim Einsetzen in die Fehlstellen dienen.

Die aus der AT397368B und der EP1792696A1 bekannten Vorrichtungen weisen den Nachteil auf, dass die Markierungen der Holzplättchen nur ungenau erkannt werden können. Somit ist eine sichere Ausrichtung der Holzplättchen nicht gewährleistet.

Aus der EP1982808B1 ist eine Vorrichtung bekannt, die zum Ausbessern von Fehlstellen in einem Holzwerkstück ausgebildet ist. Die Vorrichtung ist als mehrachsiger Roboter ausgebildet und weist einen Roboterkopf auf, wobei der Roboterkopf ein Fräswerkzeug und ein Magazin für Flicken, insbesondere Holzflicken aufweist. Weiters weist die Vorrichtung Mittel zur Einpressung des Flickens auf, die durch den Roboterkopf und/oder den Roboter gebildet sind.

Aufgabe der vorliegenden Erfindung war es, die Nachteile des Standes der Technik zu überwinden und eine verbesserte Vorrichtung bzw. ein verbessertes Verfahren zum Ausrichten der Ausbesserungsflicken zur Verfügung zu stellen.

Diese Aufgabe wird durch eine Vorrichtung und ein Verfahren gemäß den Ansprüchen gelöst.

Erfindungsgemäß ist eine Ausbesserungsvorrichtung zum Ausbesseren einer Fehlstelle in einem Holzwerkstück ausgebildet. Die Ausbesserungsvorrichtung umfasst:
- einen Setzstempel zum Einsetzen eines Ausbesserungsflickens in eine im Holzwerkstück ausgenommene Fehlstelle;
- eine Zuführvorrichtung zum Zuführen der Ausbesserungsflicken zum Setzstempel;
- ein Erfassungsmittel zum Erfassen der Orientierung des Ausbesserungsflickens mithilfe einer am Ausbesserungsflicken angeordneten Markierung;

Weiters ist eine Drehvorrichtung ausgebildet, welche zum Drehen des Ausbesserungsflickens um eine Rotationsachse dient. Das Erfassungsmittel weist einen ersten Sensor und einen zweiten Sensor auf, welche derart an der Ausbesserungsvorrichtung angeordnet sind, dass sie zum Erfassen der am Ausbesserungsflicken angeordneten Markierung an voneinander distanzierten Erfassungsbereichen dienen.

Die erfindungsgemäße Ausbesserungsvorrichtung weist den Vorteil auf, dass die Ausbesserungsflicken mit erhöhter Genauigkeit bzw. gleichzeitig auch mit erhöhter Geschwindigkeit ausgerichtet werden können und somit die Effizienz der Ausbesserungsvorrichtung gesteigert werden kann. Insbesondere wird durch die verbesserte Ausbesserungsvorrichtung gleichzeitig die Fehleranfälligkeit minimiert, wodurch gleichzeitig die Qualität der mit der Ausbesserungsvorrichtung ausgebesserten Holzwerkstücke verbessert werden kann. Insbesondere die Verwendung von zwei Sensoren bringt den Vorteil mit sich, dass die Markierung eindeutig als solche erkannt werden kann und etwaige Farbanomalien am Holzflicken nicht zu einer falschen Orientierung des Holzflickens führt.

Weiters kann es zweckmäßig sein, wenn die Drehvorrichtung einen drehbaren Setzstempel umfasst, welcher mitsamt dem Ausbesserungsflicken drehbar ist. Dies bringt den Vorteil mit sich, dass der Ausbesserungsflicken nicht relativ zum Setzstempel verdreht werden muss, sondern dass der Ausbesserungsflicken mitsamt dem Setzstempel verdreht wird und somit eine hochgenaue Winkelausrichtung des Ausbesserungsflickens ermöglicht wird.

Ferner kann vorgesehen sein, dass die Drehvorrichtung ein am drehbaren Setzstempel angeordnetes Zahnrad umfasst, welches mittels einem Zugmittel mit einem an einem Drehantrieb angeordneten Ritzel gekoppelt ist. Mittels einer derartigen Kopplung des Drehantriebes mit dem Setzstempel kann eine hochgenaue Winkelpositionierung des Setzstempels erreicht werden.

Darüber hinaus kann vorgesehen sein, dass der Drehantrieb in Form eines Servomotors ausgebildet ist. Von Vorteil ist, dass mit einem Servomotor ein schneller Drehvorgang des Setzstempels ermöglicht wird und gleichzeitig eine hohe Winkelgenauigkeit bei der Positionierung erreicht wird.

Vorteilhaft ist auch eine Ausprägung, gemäß welcher vorgesehen sein kann, dass der Setzstempel eine Flickenaufnahmefläche aufweist, welche mit Unterdruck beaufschlagbar ist. Durch diese Maßnahme kann der Ausbesserungsflicken einfach und ohne zusätzliche Hilfsmittel am Setzstempel aufgenommen werden.

Gemäß einer Weiterbildung ist es möglich, dass der Setzstempel mittels einem Betätigungsaktor, insbesondere einem Pneumatikzylinder, in Axialrichtung der Rotationsachse verschiebbar ist. Ein Pneumatikzylinder weist den Vorteil auf, dass der Setzstempel mit einer hohen Axialgeschwindigkeit verstellt werden kann und gleichzeitig die auf den Ausbesserungsflicken einwirkende Druckkraft gut vorherbestimmt bzw. eingestellt werden kann.

Ferner kann es zweckmäßig sein, wenn das Erfassungsmittel derart relativ zum Setzstempel positioniert ist, dass eine Unterseite des Ausbesserungsflickens erfassbar ist. Dies bringt den Vorteil mit sich, dass die Unterseite des Ausbesserungsflickens markiert werden kann. Somit ist nach dem Einsetzen des Ausbesserungsflickens die Markierung nicht mehr sichtbar bzw. muss das Holzwerkstück nicht mehr nachbearbeitet werden.

Darüber hinaus kann vorgesehen sein, dass der erste Sensor und der zweite Sensor als optisches Erfassungsmittel, insbesondere als Lasersensor, ausgebildet sind. Besonders eine mit optischen Sensoren erfassbare Markierung ist einfach auf den Ausbesserungsflicken aufzubringen.

Weiters kann vorgesehen sein, dass ein hohlzylindrischer Niederhaltestempel ausgebildet ist, wobei der Setzstempel koaxial zum Niederhaltestempel ausgerichtet ist und innerhalb des Hohlraums des Niederhaltestempels aufgenommen ist, wobei der Niederhaltestempel mittels einem Federelement in axialer Richtung zur Flickenaufnahmefläche des Niederhaltestempels hin vorgespannt ist, sodass er im Ruhezustand den Setzstempel überragt und ein Aufnahmeraum für den Ausbesserungsflicken ausgebildet ist. Mittels dem Niederhaltestempel kann während dem Einsetzvorgang des Ausbesserungsflickens das Holzwerkstück in seiner Position gehalten werden.

Weiters ist ein Verfahren zum Ausbesseren einer Fehlstelle in einem Holzwerkstück unter Verwendung einer Ausbesserungsvorrichtung vorgesehen. Dass Verfahren umfasst die Verfahrensschritte:
- Zuführen eines Ausbesserungsflickens zu einem Setzstempel;
- Drehen des Ausbesserungsflickes um eine Rotationsachse unter gleichzeitigem erfassen einer mit einer Markierung versehenen Oberfläche des Ausbesserungsflickens mittels einem Erfassungsmittel, wobei die Markierung die Faserrichtung des Ausbesserungsflickens kennzeichnet. Weiters erfassen ein erster Sensor und ein zweiter Sensor des Erfassungsmittels die Oberfläche des Ausbesserungsflickens an zwei voneinander distanzierten Erfassungsbereichen, wobei die Faserrichtung des Ausbesserungsflickens dann bestimmt werden kann, wenn beide Sensoren die am Ausbesserungsflicken angeordnete Markierung erfassen.

Durch das erfindungsgemäße Verfahren können Fehler bei der Orientierung der Ausbesserungsflicken weitgehend hintangehalten werden, wodurch die Ausbesserungsvorrichtung verbessert werden kann.

Entsprechend einer vorteilhaften Weiterbildung kann vorgesehen sein, dass die Sensoren des Erfassungsmittels derart an der Ausbesserungsvorrichtung angeordnet sind, dass der Ausbesserungsflicken nach Erfassung der Markierung noch um einen vorbestimmten Winkelbereich weitergedreht wird, bis die Endlage des Ausbesserungsflickens erreicht ist. Durch diese Maßnahme kann erreicht werden, dass die Drehbewegung des Ausbesserungsflickens nicht abrupt gestoppt werden muss, wenn die Markierung detektiert wird, sondern dass noch ein gewisser Nachlauf zur Verfügung steht, in welchem die Drehbewegung gestoppt werden kann.

Insbesondere kann es vorteilhaft sein, wenn die Markierung an einer Unterseite des Ausbesserungsflickens angeordnet wird.

Ferner kann vorgesehen sein, dass die Markierung parallel zur Faserrichtung am Ausbesserungsflicken angeordnet wird. Dies bringt den Vorteil mit sich, dass die Markierung am Flickenrohling noch vor dem Ausschneiden des Ausbesserungsflickens angebracht werden kann. Hierzu kann mittels einer Markiervorrichtung entlang der Faserrichtung des Ausbesserungsflickens gefahren werden, um den Ausbesserungsflickenrohling zu markieren. Durch die beschriebene Maßnahme müssen nicht die einzelnen Ausbesserungsflicken mit einer Markierung versehen werden.

Darüber hinaus kann vorgesehen sein, dass die Markierung in Form eines quer über die Unterseite des Ausbesserungsflickens verlaufenden Striches, insbesondere mittels einem Tintenstrahldrucker, auf den Ausbesserungsflicken aufgebracht wird.

In einer Alternativvariante kann auch vorgesehen sein, dass die Markierung in Form einer Leuchtfarbe auf den Ausbesserungsflicken aufgebracht wird, welche beispielsweise nur unter der Betrachtung unter einer UV-Lampe sichtbar ist.

Vorteilhaft ist auch eine Ausprägung, gemäß welcher vorgesehen sein kann, dass die Markierung am Ausbesserungsflicken angebracht wird, bevor dieser aus einem Flickenrohling ausgeschnitten wird. Durch die beschriebene Maßnahme müssen nicht die einzelnen Ausbesserungsflicken mit einer Markierung versehen werden, sondern kann zeitsparend der Flickenrohling in einem Arbeitsschritt markiert werden.

Gemäß einer Weiterbildung ist es möglich, dass die Erfassungsbereiche der Sensoren bezüglich der Rotationsachse diametral gegenüberliegend angeordnet sind. Dies bringt den Vorteil mit sich, dass durch diese Maßnahme zusätzlich überprüft werden kann, ob die Markierung korrekt am Ausbesserungsflicken angebracht ist bzw. ober der Ausbesserungsflicken zentral am Setzstempel angeordnet ist.

In einer weiteren Ausführungsvariante kann auch vorgesehen sein, dass die Sensoren bzw. deren Erfassungsbereiche nicht diametral gegenüberliegend angeordnet sind, sondern in einem Winkel zueinander versetzt angeordnet sind. Die Erfassung der Markierung mittels der Sensoren erfolgt hierbei Zeit- bzw. Winkelversetzt. Die richtige Winkellage kann hierbei errechnet werden.

Weiters kann vorgesehen sein, dass der Setzstempel relativ zu einem Maschinenrahmen verschoben wird, um diesen über der Fehlstelle in einem Holzwerkstück zu platzieren.

Darüber hinaus kann vorgesehen sein, dass das Holzwerkstück relativ zu einem Maschinenrahmen verschoben wird. Der Setzstempel kann hierbei zur Verschiebung des Holzwerkstückes aufsynchronisiert sein.

Weiters kann vorgesehen sein, dass an der Unterseite des Ausbesserungsflickens eine Schicht angeordnet ist, an welcher die Markierung aufgebracht ist. Diese Schicht kann beispielsweise durch ein Papier, einen Karton, oder ein Furnier gebildet sein.

Zum besseren Verständnis der Erfindung wird diese anhand der nachfolgenden Figuren näher erläutert.

Es zeigen jeweils in stark vereinfachter, schematischer Darstellung:
- Fig. 1: ein erstes Ausführungsbeispiel einer Ausbesserungsvorrichtung;
- Fig. 2: ein erstes Ausführungsbeispiel eines Setzstempels in einer Aufnahmeposition;
- Fig. 3: eine Verfahrensabfolge zum Ausrichten eines Ausbesserungsflickens;
- Fig. 4: Darstellungen von möglichen Fehlern an Ausbesserungsflicken;
- Fig. 5: Darstellungen von möglichen Markierungsformen an Ausbesserungsflicken;
- Fig. 6: das erste Ausführungsbeispiel des Setzstempels in einer niedergefahrenen Position;
- Fig. 7: das erste Ausführungsbeispiel des Setzstempels in einer niedergefahrenen Position;
- Fig. 8: eine schematische Darstellung einer Ausbesserungsvorrichtung mit einer Zuführvorrichtung für die Ausbesserungsflicken;
- Fig. 9: ein weiteres Ausführungsbeispiel der Sensorenanordnung.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese Lageangaben bei einer Lageänderung sinngemäß auf die neue Lage zu übertragen.

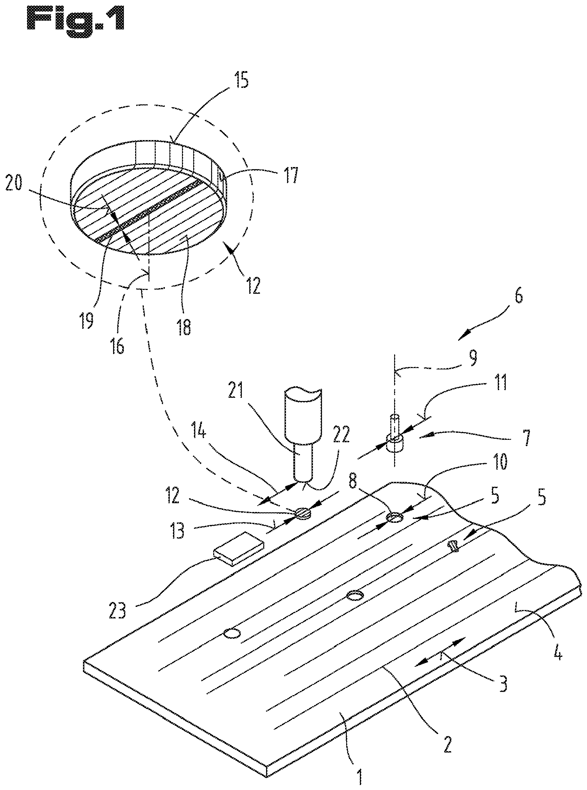

Fig. 1 zeigt eine schematische Darstellung eines Holzwerkstückes 1, welches Holzfasern 2 aufweist, die in einer Faserrichtung 3 angeordnet sind. Die Holzfasern 2 bzw. die Faserrichtung 3 des Holzwerkstückes 1 sind an einer Oberfläche 4 des Holzwerkstückes 1 besonders gut sichtbar. Da Holz ein Naturwerkstoff ist, können an der Oberfläche 4 des Holzwerkstückes 1 auch Fehlstellen 5 auftreten, welche sich optisch vom Rest der Oberfläche 4 des Holzwerkstückes 1 abheben. Derartige Fehlstellen 5 sind oft unerwünscht und müssen daher ausgebessert werden.

Die vorliegende Erfindung betrifft eine Ausbesserungsvorrichtung 6, welche in Fig. 1 rudimentär dargestellt ist.

Wie aus Fig. 1 ersichtlich, kann vorgesehen sein, dass in einem ersten Verfahrensschritt die Fehlstellen 5 mit einem Fräser 7 ausgefräst werden, wodurch eine Ausnehmung 8 in die Oberfläche 4 des Holzwerkstückes 1 eingebracht wird. Insbesondere kann vorgesehen sein, dass die Ausnehmung 8 als Sackloch ausgebildet ist. Weiters kann auch vorgesehen sein, dass die Ausnehmung 8 als Durchgangsloch ausgebildet ist.

Insbesondere kann vorgesehen sein, dass zum Herstellen der Ausnehmung 8 der Fräser 7 ausschließlich entlang seiner Fräsachse 9 verschoben wird, wodurch ein Durchmesser 10 der Ausnehmung 8 gleich groß wird wie ein Durchmesser 11 des Fräsers 7.

Alternativ dazu kann der Fräser 7 auch in einer Kreisbewegung um die Fräserachse 9 geführt werden, sodass ein Durchmesser 10 der Ausnehmung 8 größer ist als der Durchmesser 11 des Fräsers 7.

In einem weiteren Verfahrensschritt wird mittels der Ausbesserungsvorrichtung 6 ein Ausbesserungsflicken 12 in die Ausnehmung 8 eingesetzt. Der Ausbesserungsflicken 12 weist einen Durchmesser 13 auf, der dem Durchmesser 10 der Ausnehmung 8 unter Einhaltung von Fertigungstoleranzen angepasst ist, wodurch der Ausbesserungsflicken 12 in der Ausnehmung 8 aufgenommen werden kann. Weiters kann auch vorgesehen sein, dass der Ausbesserungsflicken 12 und/oder die Ausnehmung 8 konisch ausgebildet sind.

Der Ausbesserungsflicken 12 weist ebenfalls Holzfasern 2 auf, welche in einer Faserrichtung 14 des Ausbesserungsflickens 12 angeordnet sind. Die Faserrichtung 14 des Ausbesserungsflickens 12 ist an einer Oberfläche 15 des Ausbesserungsflickens 12 gut ersichtlich. Im verbauten Zustand des Ausbesserungsflickens 12 ist es gewünscht, dass die Oberfläche 15 des Ausbesserungsflickens 12 und die Oberfläche 4 des Holzwerkstückes 1 eine optisch homogen erscheinende Oberfläche bilden. Somit ist es erforderlich, dass die Faserrichtung 14 des Ausbesserungsflickens 12 mit der Faserrichtung 3 des Holzwerkstückes 1 überein stimmt. Dies kann durch die erfindungsgemäßen Maßnahmen erreicht werden.

Um in der Ausnehmung 8 einsetzbar zu sein, ist der Ausbesserungsflicken 12 zylinderförmig ausgebildet.

Zur besseren Darstellung wird der Ausbesserungsflicken 12 in Fig. 1 in einer Detailansicht vergrößert dargestellt, wobei der Ausbesserungsflicken 12 in einer anderen Ansichtsperspektive dargestellt ist. Wie aus dieser Detailansicht ersichtlich, ist der Ausbesserungsflicken 12 um eine Mittelachse 16 rotationssymmetisch ausgebildet, wobei die Mittelachse 16 eine Mantelfläche 17 koaxial umgibt. Der Oberfläche 15 des Ausbesserungsflickens 12 gegenüberliegend angeordnet ist eine Unterseite 18. An der Unterseite 18 des Ausbesserungsflickens 12 können die Holzfasern 2 ebenfalls sichtbar sein.

Wie aus der Detailansicht weiters ersichtlich, kann vorgesehen sein, dass an der Unterseite 18 des Ausbesserungsflickens 12 eine Markierung 19 angebracht ist, mittels welcher die Faserrichtung 14 des Ausbesserungsflickens 12 markiert bzw. in weiterer Folge bestimmt werden kann. Die Markierung 19 wird vorzugsweise schon beim Herstellvorgang des Ausbesserungsflickens 12 an diesem angebracht.

Wie aus der Detailansicht der Fig. 1 ersichtlich, kann vorgesehen sein, dass die Markierung 19 die Form eines Striches aufweist, welcher an der Unterseite 18 des Ausbesserungsflickens 12 angebracht ist. Die Markierung 19 verläuft hierbei vorzugsweise exakt durch die Mittelachse 16 des Ausbesserungsflickens 12. Eine Breite 20 der Markierung 19 wird hierbei möglichst gering gewählt, wobei sie jedoch so groß gewählt wird, dass die Markierung 19 für die Ausrichtung des Ausbesserungsflickens 12 in weiterer Folge noch erfassbar ist.

In einem Ausführungsbeispiel kann vorgesehen sein, dass die Markierung 19 beispielsweise mittels einem Tintenstrahldrucker auf die Unterseite 18 des Ausbesserungsflickens 12 aufgebracht wird. Natürlich können auch sonstige bekannte Mittel zur Aufbringung der Markierung 19 auf die Unterseite 18 des Ausbesserungsflickens 12 verwendet werden. Beispielsweise kann vorgesehen sein, dass die Markierung 19 mittels einem Laser in die Unterseite 18 des Ausbesserungsflicken 12 eingebrannt wird.

Die Ausbesserungsflicken 12 können beispielsweise mittels einem Kronenfräser aus einem Ausbesserungsflickenrohling herausgetrennt werden. Insbesondere kann vorgesehen sein, dass die Markierung 19 vor dem Heraustrennen des Ausbesserungsflickens 12 aus dem Flickenrohling auf die Unterseite 18 des Ausbesserungsflickens 12 aufgebracht wird.

Weiters ist in Fig. 1 schematisch ein Setzstempel 21 dargestellt, welcher zum Einsetzen bzw. Einpressen des Ausbesserungsflickens 12 in die Ausnehmung 8 dient. Der Stempel 21 weist hierbei eine Flickenaufnahmefläche 22 auf, welche zumindest während dem Einpressvorgang des Ausbesserungsflickens 12 in die Ausnehmung 8 an der Oberfläche 15 des Ausbesserungsflickens 12 anliegt.

In Fig. 1 ist schematisch auch eine Zuführvorrichtung 23 dargestellt, mittels welcher die Ausbesserungsflicken 12 zum Setzstempel 21 zugeführt werden können. Alternativ können die Ausbesserungsflicken 12 mittels dem Setzstempel 21 von einer Bereitstellungsposition übernommen werden.

Insbesondere kann vorgesehen sein, dass der Setzstempel 21 relativ zum Maschinenrahmen und somit relativ zum Holzwerkstück 1 verschiebbar ist. Weiters kann auch vorgesehen sein, dass das Holzwerkstück 1 ebenfalls relativ zum Maschinenrahen verschiebbar ist. Dadurch kann der notwendige Verschiebeweg des Setzstempels 21 möglichst gering gehalten werden.

In einem ersten Ausführungsbeispiel kann vorgesehen sein, dass der Fräser 7 und der Setzstempel 21 an einem gemeinsamen Bearbeitungskopf angeordnet sind und gemeinsam relativ zum Maschinenrahmen verschoben werden können. Dadurch kann die Positionsgenauigkeit des Fräsers 7 relativ zum Setzstempel 21 erhöht werden.

In einem weiteren Ausführungsbeispiel kann auch vorgesehen sein, dass der Fräser 7 und der Setzstempel 21 unabhängig voneinander relativ zum Maschinenrahmen verschoben werden können. Dadurch kann die Bearbeitungszeit verkürzt werden, da der Fräser 7 und der Setzstempel 21 parallel und unabhängig voneinander betrieben werden können.

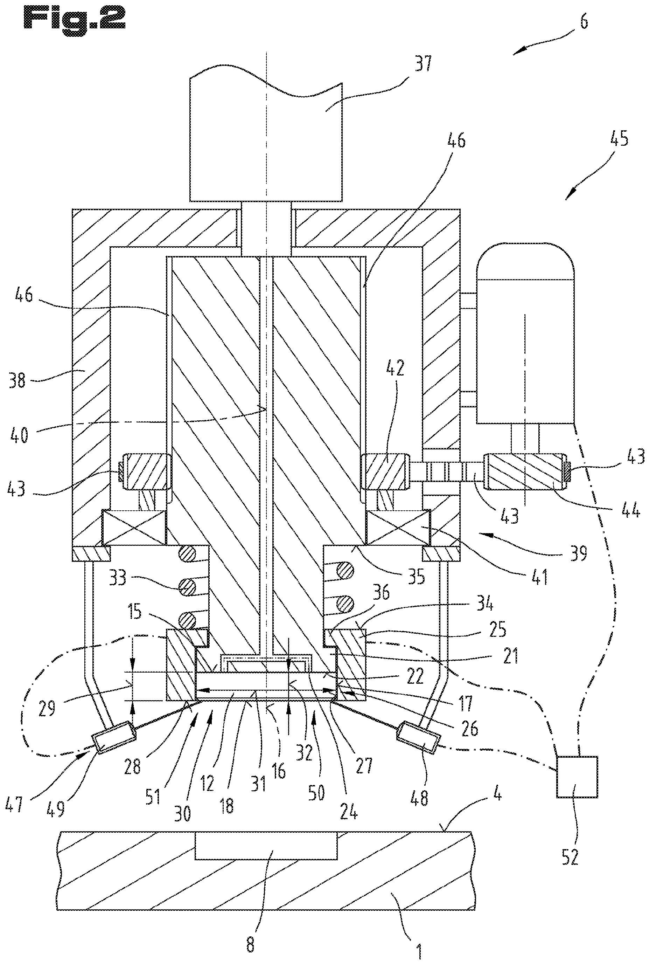

Fig. 2 zeigt eine schematische Darstellung eines Ausführungsbeispiels der Ausbesserungsvorrichtung 6, wobei insbesondere der Setzstempel 21 in einer Schnittansicht dargestellt ist. Der Setzstempel 21 ist hierbei oberhalb der Oberfläche 4 des Holzwerkstückes 1 positioniert und hat bereits einen Ausbesserungsflicken 12 aufgenommen. Die Oberfläche 15 des Ausbesserungsflickens 12 liegt hierbei an der Flickenaufnahmefläche 22 des Setzstempels 21 an.

Um den Ausbesserungsflicken 12 am Setzstempel 21 halten zu können, kann vorgesehen sein, dass die Flickenaufnahmefläche 22 Unterdruckbohrungen 24 aufweist, mittels welchen an der Flickenaufnahmefläche 22 ein Unterdruck aufgebracht werden kann, wodurch der Ausbesserungsflicken 12 an der Flickenaufnahmefläche 22 angesaugt wird.

Wie aus Fig. 2 weiters ersichtlich, kann vorgesehen sein, dass ein Niederhaltestempel 25 ausgebildet ist, welcher eine hohlzylindrische Formgebung aufweist und den Setzstempel 21 zumindest in dessen vorderen Bereich koaxial umgibt. Der Niederhaltestempel 25 weist einen Hohlraum 26 auf, in welchem der Setzstempel 21 aufgenommen ist. Der Hohlraum 26 weist eine Innenmantelfläche 27 auf, welche am Setzstempel 21 anliegt bzw. an diesem geführt ist. Weiters kann vorgesehen sein, dass im dargestellten Startzustand eine Druckfläche 28 des Niederhaltestempels 25 um einen Überstand 29 gegenüber der Flickenaufnahmefläche 22 des Setzstempels 21 vorsteht. Dadurch kann ein Aufnahmeraum 30 gebildet werden, in welchem der Ausbesserungsflicken 12 aufgenommen werden kann. Der Aufnahmeraum 30 wird durch die Innenmantelfläche 27 des Niederhaltestempels 25 und durch die Flickenaufnahmefläche 22 des Setzstempels 21 begrenzt.

Insbesondere kann vorgesehen sein, dass ein Durchmesser 31 der Innenmantelfläche 27 des Niederhaltestempels 25 geringfügig größer ist als der Durchmesser 13 des Ausbesserungsflickens 12. Dadurch kann erreicht werden, dass der Ausbesserungsflicken 12 problemlos im Aufnahmeraum 30 aufgenommen werden kann. Weiters kann vorgesehen sein, dass der Überstand 29 in etwa gleich groß ist wie eine Flickenhöhe 32.

Weiters kann vorgesehen sein, dass der Niederhaltestempel 25 mittels einem Federelement 33 in Richtung zur Oberfläche 4 des Holzwerkstückes 1 vorgespannt ist und entgegen der Vorspannung des Federelementes 33 bei Aufbringung einer Kraft auf die Druckfläche 28 relativ zum Setzstempel 21 verschiebbar ist. Insbesondere kann vorgesehen sein, dass das Federelement 33 zwischen dem Setzstempel 21 und dem Niederhaltestempel 25 angeordnet ist bzw. sich an diesem abstützt. Beispielsweise kann vorgesehen sein, dass am Niederhaltestempel 25 der Druckfläche 28 gegenüberliegend eine Abstützfläche 34 angeordnet ist an welcher das Federelement 33 anliegt. Weiters kann am Setzstempel 21 ebenfalls eine Abstützfläche 35 ausgebildet sein, welche der Abstützfläche 34 des Niederhaltestempels 25 zugewandt ist. Das Federelement 33 kann sich somit zwischen der Abstützfläche 34 des Niederhaltestempels 25 und der Abstützfläche 35 des Setzstempels 21 erstrecken.

Wie aus Fig. 2 ersichtlich, kann der Niederhaltestempel 25 derart am Setzstempel 21 angeordnet sein, dass er in Axialrichtung relativ zum Setzstempel 21 verschiebbar ist. Weiters kann ein Anschlagelement 36 ausgebildet sein, welches die axiale Verschiebung des Niederhaltestempels 25 relativ zum Setzstempels 21 in Richtung Oberfläche 4 des Holzwerkstückes 1 begrenzt. Die Positionierung des Anschlagelements 36 bestimmt den Wert des Überstandes 29.

Wie aus dem Ausführungsführungsbeispiel aus Fig. 2 ersichtlich, kann vorgesehen sein, dass das Anschlagelement 36 in Form eines Absatzes ausgebildet ist.

Um einen dargestellten Niederhaltestempel 25 auf den Setzstempel 21 aufbringen zu können, kann vorgesehen sein, dass der Niederhaltestempel 25 entlang einer Mittelebene in zwei Hälften geteilt ist, welche mittels Befestigungsmittel zusammengehalten werden.

Alternativ dazu kann vorgesehen sein, dass der Setzstempel 21 einen Kopfteil aufweist, welcher mittels einem Befestigungselement mit dem Hauptteil verbindbar ist, wobei zwischen dem Kopfteil und dem Hauptteil der Absatz ausgebildet ist.

Weiters ist ein Betätigungsaktor 37 vorgesehen, mittels welchem der Setzstempel 21 in Axialrichtung zum Holzwerkstück 1 hin verschoben werden kann. Insbesondere kann vorgesehen sein, dass eine Setzstempelhalterung 38 ausgebildet ist, welche zur Aufnahme bzw. zur Führung des Setzstempels 21 dient. Der Betätigungsaktor 37 kann beispielsweise in Form eines Pneumatikzylinders ausgebildet sein. Der Pneumatikzylinder kann an der Setzstempelhalterung 38 befestigt sein, wobei eine Kolbenstange des Pneumatikzylinders mit dem Setzstempel 21 gekoppelt sein kann. Durch diese Maßnahme kann der Setzstempels 21 relativ zur Setzstempelhalterung 38 in Axialrichtung verschoben werden.

Anstatt eines Pneumatikzylinders können natürlich auch sonstige Betätigungsaktoren 37 verwendet werden. Weiters kann vorgesehen sein, dass die Setzstempelhalterung 38 und der Setzstempel 21 als Zylinder/Kolbenkombination ausgeführt sind.

Die Ausbesserungsvorrichtung 6 umfasst weiters eine Drehvorrichtung 39 mittels welcher der Ausbesserungsflicken 12 relativ zum Holzwerkstück 1 drehbar sind. Dadurch kann die Faserrichtung 14 des Ausbesserungsflickens 12 an die Faserrichtung 3 des Holzwerkstückes 1 angepasst werden. Die Drehvorrichtung 39 dreht den Ausbesserungsflicken 12 um eine Rotationsachse 40. Im Idealfall ist der Ausbesserungsflicken 12 derart in der Ausbesserungsvorrichtung 6 aufgenommen, dass die Rotationsachse 40 der Drehvorrichtung 39 und die Mittelachse 16 des Ausbesserungsflickens 12 koaxial zueinander liegen. Dadurch kann der Ausbesserungsflicken 12 mittels der Drehvorrichtung 39 um seine Mittelachse 16 gedreht werden.

Im vorliegenden Ausführungsbeispiel ist die Drehvorrichtung 39 dadurch ausgebildet, dass der Setzstempel 21 mitsamt den daran aufgenommenen Ausbesserungsflicken 12 relativ zur Setzstempelhalterung 38 und somit relativ zum Holzwerkstück 1 drehbar ist. Die Drehvorrichtung 39 kann eine Lagerung 41 aufweisen, mittels welcher der Setzstempel 21 relativ zur Setzstempelhalterung 38 um die Rotationsachse 40 verdrehbar an dieser gelagert ist. Die Lagerung 41 kann sowohl als Drehlagerung als auch als Axiallagerung ausgebildet sein, mittels welcher der Setzstempel 21 sowohl um die Rotationsachse 40 drehbar als auch entlang der Rotationsachse 40 in Axialrichtung verschiebbar an der Setzstempelhalterung 38 aufgenommen ist.

Beispielsweise kann vorgesehen sein, dass die Lagerung 41 als Gleitlagerung ausgebildet ist, durch welche die beschriebenen Bewegungen ermöglicht werden.

Weiters kann die Drehvorrichtung 39 ein Zahnrad 42 umfassen, welches mittels einem Zugmittel 43 mit einem Ritzel 44 gekoppelt sein kann. Das Ritzel 44 kann an einem Drehantrieb 45 angeordnet sein, mittels welchem die Drehung des Setzstempels 21 um die Rotationsachse 40 eingeleitet werden kann.

Wie aus Fig. 2 ersichtlich, kann vorgesehen sein, dass der Drehantrieb 45 mit der Setzstempelhalterung 38 gekoppelt ist bzw. an dieser angeordnet ist. Der Drehantrieb 45 kann beispielsweise in Form eines Elektromotors, insbesondere als Servomotor, ausgebildet sein.

Das Zugmittel 43 kann beispielsweise in Form eines Zahnriemens ausgebildet sein. Weiters ist es auch denkbar, dass beispielsweise eine Kette oder ein sonstiges Zugmittel zur Kraftübertragung zwischen dem Zahnrad 42 und dem Ritzel 44 verwendet wird.

In einem weiteren Ausführungsbeispiel ist es auch denkbar, dass das Ritzel 44 ohne dem zwischengeschaltenem Zugmittel 43 direkt in das Zahnrad 42 eingreift.

In wieder einer anderen Ausführungsvariante ist es auch denkbar, dass das Zahnrad 42 integral im Setzstempel 21 ausgebildet ist und das Ritzel 44 direkt in diesen eingreift. Bei einer derartigen Ausführungsvariante könnte eine axiale Verschiebung zwischen dem Setzstempel 21 und dem Ritzel 44 direkt ausgeglichen werden. Natürlich ist es auch denkbar, dass der Drehantrieb 45 über eine sonstige Drehmomentenverbindung mit dem Setzstempel 21 gekoppelt ist.

Im vorliegenden Ausführungsbeispiel gemäß Fig. 2 ist das Zahnrad 42 mittels der Lagerung 41 drehbar an der Setzstempelhalterung 38 aufgenommen. Weiters kann vorgesehen sein, dass im Setzstempel 21 eine oder mehrere Mitnehmernuten 46 ausgebildet sind, in welche das Zahnrad 42 eingreift. Durch die Mitnehmernuten 46 ist der Setzstempel 21 relativ zum Zahnrad 42 in Axialrichtung verschiebbar, wobei zu jedem Zeitpunkt die Drehmomentenverbindung erhalten bleibt.

Anstatt der Mitnehmernut 46 kann natürlich auch eine sonstige in Axialrichtung verschiebbare Drehmomentenverbindung, wie etwa eine Mikroverzahnung, eine Polygonverbindung oder sonstige formschlüssige Verbindungen ausgebildet sein.

Weiters ist ein Erfassungsmittel 47 vorgesehen, welches zur Erfassung der Winkellage des Ausbesserungsflickens 12 anhand der Markierung 19 dient. Das Erfassungsmittel 47 umfasst einen ersten Sensor 48 und einen zweiten Sensor 49, welche zum Beobachten der Unterseite 18 des Ausbesserungsflickens 12 in einem ersten Erfassungsbereich 50 und in einem zweiten Erfassungsbereich 51 ausgebildet sind.

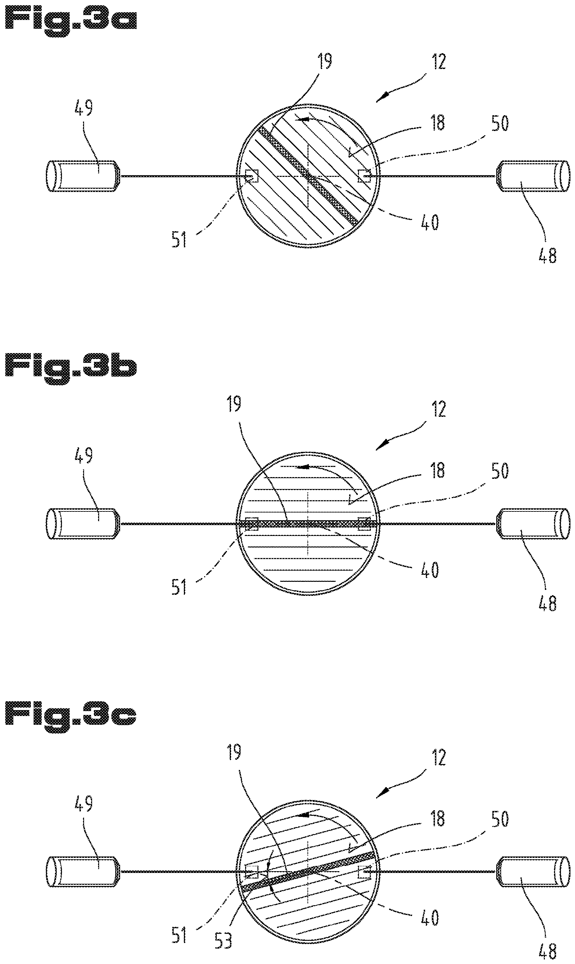

Der genaue Vorgang zur Erfassung der Winkellage wird in weiterer Folge anhand der Figuren 3a bis 3c erklärt.

Der erste Sensor 48 und der zweite Sensor 49 sind ebenfalls mit der Setzstempelhalterung 38 gekoppelt. Somit ist der Ausbesserungsflicken 12 mittels dem Drehantrieb 45 relativ zum ersten Sensor 48 bzw. zum zweiten Sensor 49 verdrehbar.

Wie aus Fig. 2 ersichtlich, kann vorgesehen sein, dass die beiden Sensoren 48, 49 bezüglich dem Setzstempel 21 einander diametral gegenüberliegend angeordnet sind. Der erste Sensor 48 bzw. der zweite Sensor 49 sowie der Drehantrieb 45 sind mit einer Steuerung 52 gekoppelt, mittels welcher die Bewegung des Drehantriebes 45 gesteuert werden kann. Dadurch kann der Ausbesserungsflicken 12 bezüglich dessen Winkellage exakt ausgerichtet werden.

Wie aus Fig. 2 ersichtlich, sind der erste Sensor 48 und der zweite Sensor 49 derart unterhalb des Setzstempels 21 angeordnet, dass die Unterseite 18 des Ausbesserungsflickens 12 mittels der Sensoren 48, 49 erfassbar ist.

In einem ersten Ausführungsbeispiel kann vorgesehen sein, dass die Markierung 19 als Farbmarkierung ausgebildet ist und dass die Sensoren 48, 49 als optische Sensoren zur Erfassung der Farbmarkierung ausgebildet sind.

In einer weiteren Ausführungsvariante kann auch vorgesehen sein, dass die Markierung 19 beispielsweise magnetische oder magnetisierbare Partikel enthält und die Sensoren 48, 49 beispielsweise in Form von Induktionssensoren ausgebildet sind.

Anhand der Fig. 3a bis 3c wird die Ausrichtung bzw. die richtige Winkeldrehung des Ausbesserungsflickens 12 beschrieben. Für die Beschreibung des Verfahrensablaufes ist in den Fig. 3a bis 3c die Ausbesserungsvorrichtung 6 bzw. der Ausbesserungsflicken 12 in einer Ansicht von unten bzw. in einer Draufschau auf die Unterseite 18 des Ausbesserungsflickens 12 dargestellt.

Wie aus Fig. 3a ersichtlich, kann der Ausbesserungsflicken 12 beliebig in der Ausbesserungsvorrichtung 6 aufgenommen werden. Anschließend wird der Ausbesserungsflicken 12 mittels der Drehvorrichtung 39 um die Rotationsachse 40 gedreht, wobei der erste Sensor 48 und der zweite Sensor 49 zwei diametral gegenüber liegende Erfassungsbereiche 50, 51 erfassen und auf das Vorhandensein der Markierung 19 kontrollieren.

Erst wenn, wie in Fig. 3b dargestellt, die Markierung 19 derart ausgerichtet ist, dass in beiden Erfassungsbereichen 50, 51 gleichzeitig die Markierung 19 detektiert wird, kann mittels der Steuerung 52 die aktuelle Winkellage der Faserrichtung 14 des Ausbesserungsflickens 12 bestimmt werden.

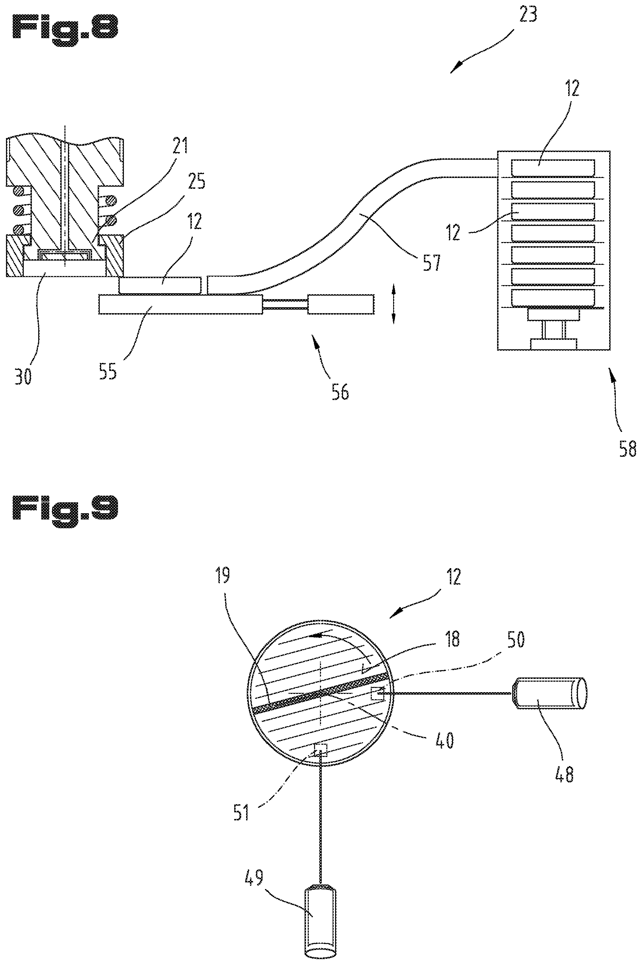

Alternativ dazu kann, wie in Fig. 9 dargestellt, auch vorgesehen sein, dass Markierung 19 derart ausgerichtet ist, bzw. die beiden Erfassungsbereiche 50, 51 derart angeordnet sind, dass die Markierung 19 in den beiden Erfassungsbereichen 50, 51 nicht gleichzeitig detektiert wird, sondern dass die Markierung 19 zuerst in einem ersten der Erfassungsbereiche 50, 51 detektiert wird und erst nach einer gewissen Winkeldrehung in einem zweiten der Erfassungsbereiche 50, 51 detektiert wird. Hierbei kann ebenfalls die Ausrichtung des Ausbesserungsflickens 12 festgestellt werden.

Um den Drehvorgang zur Ausrichtung des Ausbesserungsflickens 12 nicht abrupt stoppen zu müssen, kann vorgesehen sein, dass die Markierung 19 derart zur Faserrichtung 14 des Ausbesserungsflickens 12 angeordnet ist, dass die richtige Winkellage des Ausbesserungsflickens 12 relativ zum Holzwerkstück 1 erst in einem Nachdrehwinkel 53 erreicht wird, welcher zur Detektionslage gemäß der Fig. 3b versetzt ist. Der Nachdrehwinkel 53 wird hierbei so groß gewählt, dass die aufgrund der Massenträgheit des Setzstempels 21 auftretenden Abbremskräfte zum Stoppen der Rotationsbewegung des Setzstempels 21 nicht zu groß sind. Der Nachdrehwinkel 53 kann beispielsweise zwischen 1° und 90°, insbesondere zwischen 20° und 60°, bevorzugt zwischen 40° und 50° betragen.

Die Erfassung des aktuellen Drehwinkels des Ausbesserungsflickens 12 mittels zumindest zweier Sensoren 48, 49 bringt den Vorteil mit sich, dass etwaige Farbfehler an der Unterseite 18 des Ausbesserungsflickens 12 bzw. eine falsche Lage der Markierung 19 bzw. eine falsche Positionierung des Ausbesserungsflickens 12 am Setzstempel nicht fälschlicherweise als korrekte Lage des Ausbesserungsflickens 12 erkannt wird.

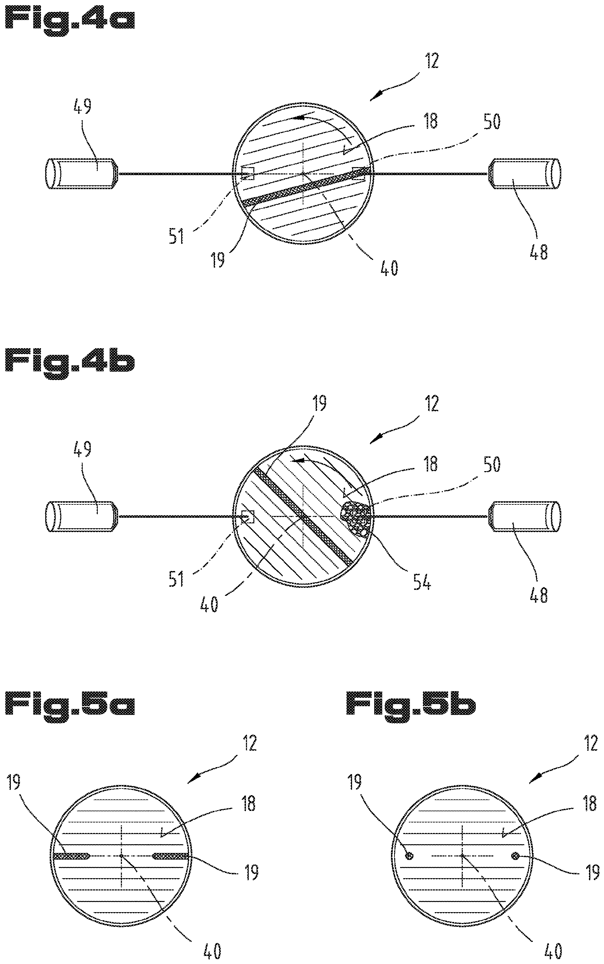

Derartige Fehler sind beispielsweise in den Fig. 4a und 4b dargestellt. Aus der Fig. 4a ist ersichtlich, dass eine außermittige Lage der Markierung 19 bzw. eine außermittige Anordnung des Ausbesserungsflickens 12 dazu führt, dass der entsprechende Ausbesserungsflicken 12 ausgeschieden wird, da die korrekte Lage nicht wie oben beschrieben ermittelt werden kann.

Wird nicht spätestens nach einer Winkeldrehung von 360° die korrekte Lage des Ausbesserungsflickens 12 erkannt, so wird in der Steuerung 52 ein entsprechender Befehl zum Ausscheiden des Ausbesserungsflickens 12 gegeben.

Ein weiteres Ausführungsbeispiel in dem die Vorteile von zwei Sensoren 48, 49 ersichtlich werden, findet sich in Fig. 4b. Wie aus der Fig. 4b ersichtlich, können an der Unterseite 18 des Ausbesserungsflickens 12 beispielsweise Farbflecken 54 auftreten, welche von den Sensoren 48, 49 fälschlicherweise nicht von der Markierung 19 unterschieden werden können. Durch die Redundanz der beiden Sensoren 48, 49 kann jedoch, wie aus Fig. 4b ersichtlich, detektiert werden, dass noch nicht die korrekte Winkellage erreicht ist, sondern ein derartiger Farbfleck 54 detektiert wird und der Ausbesserungsflicken 12 somit weiter gedreht werden muss, bis von beiden Sensoren 48, 49 gleichzeitig die Markierung 19 detektiert wird.

In dem Ausführungsbeispiel gemäß der Fig. 3 ist die Markierung 19 in Form eines durchgehenden Striches ausgebildet.

In den Fig. 5a und b sind weitere Ausführungsmöglichen der Markierung 19 dargestellt. Wie aus der Fig. 5a ersichtlich, kann vorgesehen sein, dass die Markierung 19 in Form eines unterteilten Striches ausgebildet ist.

Wie aus Fig. 5b ersichtlich, kann vorgesehen sein, dass die Markierung 19 beispielsweise durch mehrere Punkte ausgebildet ist.

Wichtig ist jedoch, dass in der korrekten Lage des Ausbesserungsflickens 12 in beiden Erfassungsbereichen 50, 51 die Markierung 19 detektiert werden kann.

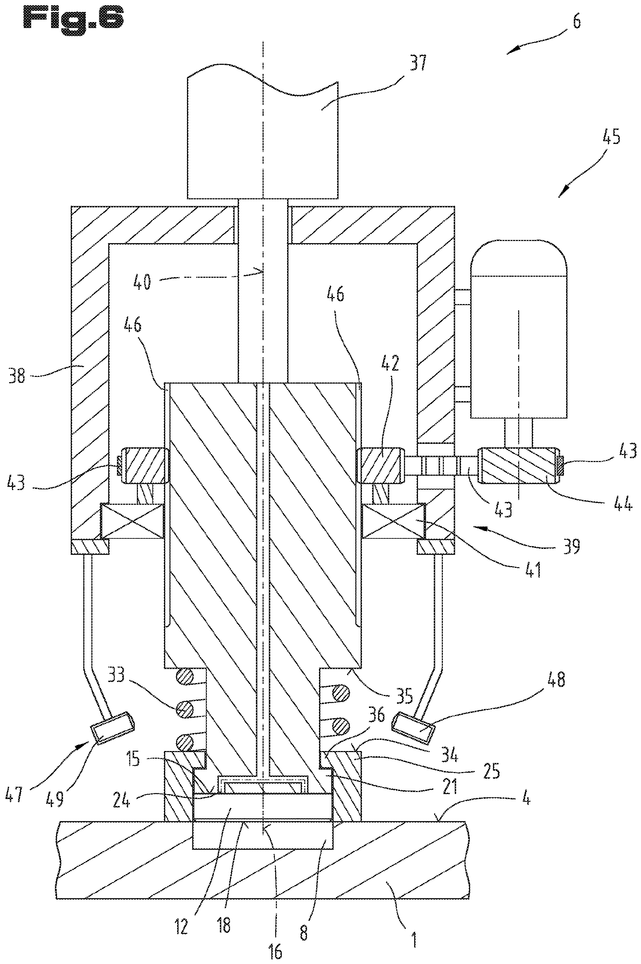

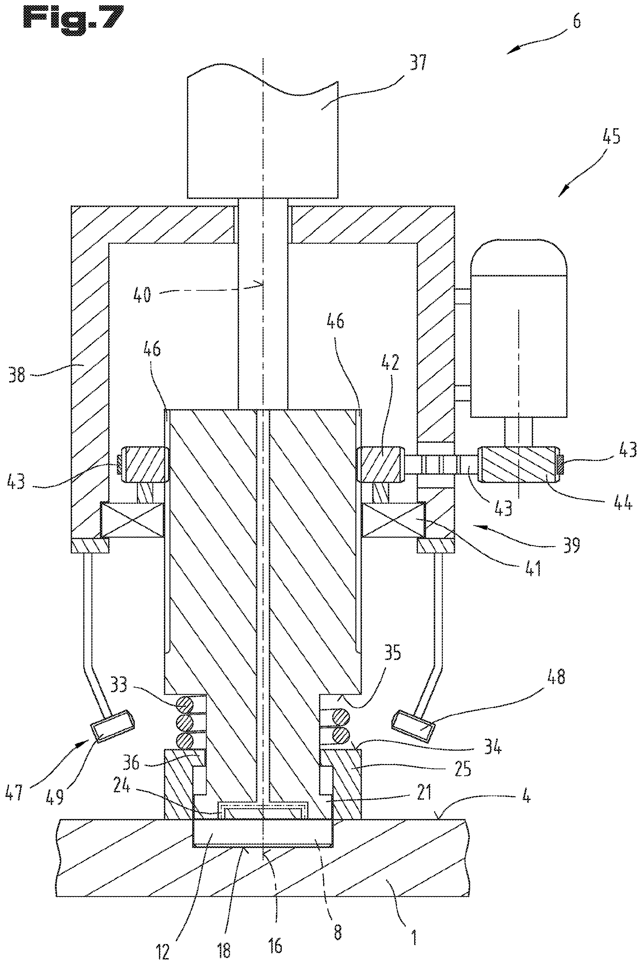

In den Figuren 6 und 7 sind weitere Verfahrensschritte zum Einsetzen des Ausbesserungsflickens 12 in die Ausnehmung 8 dargestellt, wobei wiederum für gleiche Teile gleiche Bezugszeichen bzw. Bauteilbezeichnungen wie in der vorangegangenen Fig. 2 verwendet werden. Um unnötige Wiederholungen zu vermeiden, wird auf die detaillierte Beschreibung in der vorangegangenen Fig. 2 hingewiesen bzw. Bezug genommen.

Die Verfahrensschritte gemäß Fig. 6 folgen auf die korrekte Rotationsausrichtung des Ausbesserungsflickens 12.

Wie aus Fig. 6 ersichtlich, wird der Setzstempel 21 mittels dem Betätigungsaktor 37 in Axialrichtung zum Holzwerkstück 1 verschoben, wobei entsprechend der Fig. 6 zuerst der Niederhaltestempel 25 am Holzwerkstück 1 aufliegt und bei weiterer Zustellbewegung des Setzstempels 21 mittels der Federkraft des Federelementes 33 gegen das Holzwerkstück 1 gedrückt wird.

Der Setzstempel 21 wird relativ zum Niederhaltestempel 25 weiter in Richtung Holzwerkstück 1 verschoben, bis entsprechend der Ansicht nach Fig. 7 der Ausbesserungsflicken 12 in die Ausnehmung 8 eingesetzt ist.

In weiteren, nicht dargestellten Verfahrensschritten wird der Setzstempel 21 mittels dem Betätigungsaktor 37 wieder nach oben bewegt und ein neuer Ausbesserungsflicken 12 in den Aufnahmeraum 30 eingesetzt.

In einem weiteren, nicht dargestellten Ausführungsbeispiel kann auch vorgesehen sein, dass den Setzstempel 21 kein Niederhaltestempel 25 umgibt.

Fig. 8 zeigt eine schematische Darstellung der Zuführvorrichtung 23, wobei wiederum für gleiche Teile gleiche Bezugszeichen bzw. Bauteilbezeichnungen wie in den vorangegangenen Figuren verwendet werden. Um unnötige Wiederholungen zu vermeiden, wird auf die detaillierte Beschreibung in den vorangegangenen Figuren hingewiesen bzw. Bezug genommen.

Wie aus Fig. 8 ersichtlich, kann die Zuführvorrichtung 23 einen Manipulator 55 umfassen, mittels welchem die Ausbesserungsflicken 12 in den Aufnahmeraum 30 eingesetzt werden können. Der Manipulator 55 kann mit entsprechenden Aktoren 56 zum bewegen des Manipulators 55 gekoppelt sein. Weiters kann vorgesehen sein, dass zum Zuführen der Ausbesserungsflicken 12 zum Manipulator 55 ein Förderschlauch 57 vorgesehen ist, in welchem die Ausbesserungsflicken 12 beispielsweise mittels Druckluft von einem Flickenspeicher 58 zum Manipulator 55 befördert werden können.

Fig. 9 zeigt in einer schematischen Darstellung ein weiteres Ausführungsbeispiel der Anordnung von Sensoren 48, 49. Wie aus Fig. 9 ersichtlich, kann vorgesehen sein, dass die Sensoren 48, 49 nicht diametral einander gegenüberliegend am Setzstempel 21 angeordnet sind, sondern um einen Winkel, beispielsweise 90° zueinander versetzt am Setzstempel 21 angeordnet sind. Hierbei wird die Markierung 19 zuerst von einem ersten der Sensoren 48, 49 detektiert. Erst nach einer gewissen Winkeldrehung des Ausbesserungsflickens 12 wird die Markierung 19 von einem zweiten der Sensoren 48, 49 detektiert. Über die Kenntnis der Lage der Sensoren 48, 49 bzw. die vollzogene Winkeldrehung zwischen den beiden Detektionen kann hierbei die korrekte Winkellage des Ausbesserungsflickens 12 ermittelt werden.

Die Ausführungsbeispiele zeigen mögliche Ausführungsvarianten, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Der Schutzbereich ist durch die Ansprüche bestimmt. Die Beschreibung und die Zeichnungen sind jedoch zur Auslegung der Ansprüche heranzuziehen. Einzelmerkmale oder Merkmalskombinationen aus den gezeigten und beschriebenen unterschiedlichen Ausführungsbeispielen können für sich eigenständige erfinderische Lösungen darstellen. Die den eigenständigen erfinderischen Lösungen zugrundeliegende Aufgabe kann der Beschreibung entnommen werden.

Sämtliche Angaben zu Wertebereichen in gegenständlicher Beschreibung sind so zu verstehen, dass diese beliebige und alle Teilbereiche daraus mitumfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1, oder 5,5 bis 10.

Der Ordnung halber sei abschließend darauf hingewiesen, dass zum besseren Verständnis des Aufbaus Elemente teilweise unmaßstäblich und/oder vergrößert und/oder verkleinert dargestellt wurden.

### Bezugszeichenaufstellung

| | | | |
|---|---|---|---|
| 1 | Holzwerkstück | 29 | Überstand |
| 2 | Holzfaser | 30 | Aufnahmeraum |
| 3 | Faserrichtung Holzwerkstück | 31 | Durchmesser Innenmantelfläche |
| 4 | Oberfläche Holzwerkstück | 32 | Flickenhöhe |
| 5 | Fehlstelle | 33 | Federelement |
| 6 | Ausbesserungsvorrichtung | 34 | Abstützfläche Niederhaltestempel |
| 7 | Fräser | 35 | Abstützfläche Setzstempel |
| 8 | Ausnehmung | 36 | Anschlagelement |
| 9 | Fräserachse | 37 | Betätigungsaktor |
| 10 | Durchmesser Ausnehmung | 38 | Setzstempelhalterung |
| 11 | Durchmesser Fräser | 39 | Drehvorrichtung |
| 12 | Ausbesserungsflicken | 40 | Rotationsachse |
| 13 | Durchmesser Ausbesserungsfli- cken | 41 | Lagerung |
| | | 42 | Zahnrad |
| 14 | Faserrichtung Ausbesserungsfli- cken | 43 | Zugmittel |
| | | 44 | Ritzel |
| 15 | Oberfläche Ausbesserungsflicken | 45 | Drehantrieb |
| 16 | Mittelachse | 46 | Mitnehmernut |
| 17 | Mantelfläche | 47 | Erfassungsmittel |
| 18 | Unterseite Ausbesserungsflicken | 48 | erster Sensor |
| 19 | Markierung | 49 | zweiter Sensor |
| 20 | Breite Markierung | 50 | erster Erfassungsbereich |
| 21 | Setzstempel | 51 | zweiter Erfassungsbereich |
| 22 | Flickenaufnahmefläche | 52 | Steuerung |
| 23 | Zuführvorrichtung | 53 | Nachdrehwinkel |
| 24 | Unterdruckbohrung | 54 | Farbfleck |
| 25 | Niederhaltestempel | 55 | Manipulator |
| 26 | Hohlraum | 56 | Aktor |
| 27 | Innenmantelfläche | 57 | Förderschlauch |
| 28 | Druckfläche | 58 | Flickenspeicher |

## Patentansprüche

1. Ausbesserungsvorrichtung (6) zum Ausbesseren einer Fehlstelle (5) in einem Holzwerkstück (1), umfassend:
- einen Setzstempel (21) zum Einsetzen eines Ausbesserungsflickens (12) in ein im Holzwerkstück (1) eingebrachte Ausnehmung (8);
- eine Zuführvorrichtung (23) zum Zuführen der Ausbesserungsflicken (12) zum Setzstempel (21);
- ein Erfassungsmittel (47) zum Erfassen der Orientierung des Ausbesserungsflickens (12) mithilfe einer am Ausbesserungsflicken (12) angeordneten Markierung (19); wobei
eine Drehvorrichtung (39) ausgebildet ist, welche zum Drehen des Ausbesserungsflickens (12) um eine Rotationsachse (40) dient, **dadurch gekennzeichnet daß** das Erfassungsmittel (47) einen ersten Sensor (48) und einen zweiten Sensor (49) aufweist, welche derart an der Ausbesserungsvorrichtung (6) angeordnet sind, dass sie zum Erfassen der am Ausbesserungsflicken (12) angeordneten Markierung (19) an voneinander distanzierten Erfassungsbereichen (50, 51) dienen.

2. Ausbesserungsvorrichtung nach Anspruch 1, **dadurch gekennzeichnet, dass** die Drehvorrichtung (39) einen drehbaren Setzstempel (21) umfasst, welcher mitsamt dem Ausbesserungsflicken (12) drehbar ist.

3. Ausbesserungsvorrichtung nach Anspruch 2, **dadurch gekennzeichnet, dass** die Drehvorrichtung (39) ein am drehbaren Setzstempel (21) angeordnetes Zahnrad (42) umfasst, welches mittels einem Zugmittel (43) mit einem an einem Drehantrieb (45) angeordneten Ritzel (44) gekoppelt ist.

4. Ausbesserungsvorrichtung nach Anspruch 3, **dadurch gekennzeichnet, dass** der Drehantrieb (45) in Form eines Servomotors ausgebildet ist.

5. Ausbesserungsvorrichtung nach einem der vorhergehenden Ansprüche, **dadurch gekennzeichnet, dass** der Setzstempel (21) eine Flickenaufnahmefläche (22) aufweist, welche mit Unterdruck beaufschlagbar ist.

6. Ausbesserungsvorrichtung nach einem der vorhergehenden Ansprüche, **dadurch gekennzeichnet, dass** der Setzstempel (21) mittels einem Betätigungsaktor (37), insbesondere einem Pneumatikzylinder, in Axialrichtung der Rotationsachse (40) verschiebbar ist.

7. Ausbesserungsvorrichtung nach einem der vorhergehenden Ansprüche, **dadurch gekennzeichnet, dass** das Erfassungsmittel (47) derart relativ zum Setzstempel (21) positioniert ist, dass eine Unterseite (18) des Ausbesserungsflickens (12) erfassbar ist.

8. Ausbesserungsvorrichtung nach einem der vorhergehenden Ansprüche, **dadurch gekennzeichnet, dass** der erste Sensor (48) und der zweite Sensor (49) als optisches Erfassungsmittel, insbesondere als Lasersensor, ausgebildet sind.

9. Ausbesserungsvorrichtung nach einem der vorhergehenden Ansprüche, **dadurch gekennzeichnet, dass** ein hohlzylindrischer Niederhaltestempel (25) ausgebildet ist, wobei der Setzstempel (21) koaxial zum Niederhaltestempel (25) ausgerichtet ist und innerhalb des Hohlraum (26) des Niederhaltestempels (25) aufgenommen ist, wobei der Niederhaltestempel (25) mittels einem Federelement (33) in axialer Richtung zur Flickenaufnahmefläche (22) des Niederhaltestempels hin (25) vorgespannt ist, sodass er im Ruhezustand den Setzstempel (21) überragt und ein Aufnahmeraum (30) für den Ausbesserungsflicken (12) ausgebildet ist.

10. Verfahren zum Ausbesseren einer Fehlstelle (5) in einem Holzwerkstück (1) unter Verwendung einer Ausbesserungsvorrichtung (6), insbesondere einer Ausbesserungsvorrichtung (6) nach einem der vorhergehenden Ansprüche, umfassend die Verfahrensschritte:
- Zuführen eines Ausbesserungsflickens (12) zu einem Setzstempel (21);
- Drehen des Ausbesserungsflickes um eine Rotationsachse (40) unter gleichzeitigem erfassen einer mit einer Markierung (19) versehenen Oberfläche des Ausbesserungsflickens (12) mittels einem Erfassungsmittel (47), wobei die Markierung (19) die Faserrichtung (14) des Ausbesserungsflickens (12) **kennzeichnet,**
**dadurch** gekennzeichnet, dass
das ein erster Sensor (48) und ein zweiter Sensor (49) des Erfassungsmittels (47) die Oberfläche des Ausbesserungsflickens (12) an zwei voneinander distanzierten Erfassungsbereichen (50, 51) erfassen, wobei die Faserrichtung (14) des Ausbesserungsflickens (12) dann bestimmt werden kann, wenn beide Sensoren (48, 49) die am Ausbesserungsflicken (12) angeordnete Markierung (19) erfassen.

11. Verfahren nach Anspruch 10, **dadurch gekennzeichnet, dass** die Sensoren (48, 49) des Erfassungsmittels (47) derart an der Ausbesserungsvorrichtung (6) angeordnet sind, dass der Ausbesserungsflicken (12) nach Erfassung der Markierung (19) noch um einen vorbestimmten Winkelbereich weitergedreht wird, bis die Endlage des Ausbesserungsflickens (12) erreicht ist.

12. Verfahren nach Anspruch 10 oder 11, **dadurch gekennzeichnet, dass** die Markierung (19) an einer Unterseite (18) des Ausbesserungsflickens (12) angeordnet wird.

13. Verfahren nach einem der Ansprüche 10 bis 12, **dadurch gekennzeichnet, dass** die Markierung (19) parallel zur Faserrichtung (14) am Ausbesserungsflicken (12) angeordnet wird.

14. Verfahren nach einem der Ansprüche 10 bis 13, **dadurch gekennzeichnet, dass** die Markierung (19) in Form eines quer über die Unterseite (18) des Ausbesserungsflickens (12) verlaufenden Striches, insbesondere mittels einem Tintenstrahldrucker, auf den Ausbesserungsflicken (12) aufgebracht wird.

15. Verfahren nach Anspruch 14, **dadurch gekennzeichnet, dass** die Markierung (19) am Ausbesserungsflicken (12) angebracht wird, bevor dieser aus einem Flickenrohling ausgeschnitten wird.

16. Verfahren nach Anspruch 15, **dadurch gekennzeichnet, dass** die Erfassungsbereiche (50, 51) der Sensoren (48, 49) bezüglich der Rotationsachse (40) diametral gegenüberliegend angeordnet sind.

17. Verfahren nach einem der Ansprüche 10 bis 16, **dadurch gekennzeichnet, dass** der Setzstempel (21) relativ zu einem Maschinenrahmen verschoben wird, um diesen über der Fehlstelle (5) in einem Holzwerkstück (1) zu platzieren.

18. Verfahren nach einem der Ansprüche 10 bis 17, **dadurch gekennzeichnet, dass** das Holzwerkstück (1) relativ zu einem Maschinenrahmen verschoben wird.

## Claims

1. A repair device (6) for repairing a defect (5) in a wooden workpiece (1), comprising:
- a setting punch (21) for inserting a repair patch (12) into a recess (8) formed in a wooden workpiece (1);
- a feed device (23) for feeding the repair patches (12) to the setting punch (21);
- a detection means (47) for detecting the orientation of the repair patch (12) with the aid of a marking (19) arranged on the repair patch (12);
wherein
a rotary device (39) is provided, which is used for rotating the repair patch (12) about a rotational axis (40), **characterized in that**
the detection means (47) comprises a first sensor (48) and a second sensor (49), which are arranged at the repair device (6) in such a way that they are used for detecting the marking (19) arranged on the repair patch (12), at detection regions (50, 51) arranged at a distance from one another.

2. The repair device according to claim 1, **characterized in that** the rotary device (39) comprises a rotatable setting punch (21), which is rotatable together with the repair patch (12).

3. The repair device according to claim 2, **characterized in that** the rotary device (39) comprises a gear wheel (42) arranged at the rotatable setting punch (21), which gear wheel (42) is coupled, by means of a traction means (43), to a pinion (44) arranged at a rotary drive (45).

4. The repair device according to claim 3, **characterized in that** the rotary drive (45) is configured as a servomotor.

5. The repair device according to one of the preceding claims, **characterized in that** the setting punch (21) comprises a patch receiving surface (22), which is impingeable with negative pressure.

6. The repair device according to one of the preceding claims, **characterized in that** the setting punch (21) is displaceable in the axial direction of the rotational axis (40) by means of an activation actuator (37), in particular a pneumatic cylinder.

7. The repair device according to one of the preceding claims, **characterized in that** the detection means (47) is positioned relative to the setting punch (21) in such a way that a bottom side (18) of the repair patch (12) is detectable.

8. The repair device according to one of the preceding claims, **characterized in that** the first sensor (48) and the second sensor (49) are configured as an optical detection means, in particular as a laser sensor.

9. The repair device according to one of the preceding claims, **characterized in that** a hollow-cylindrical holding-down punch (25) is provided, wherein the setting punch (21) is aligned coaxially to the holding-down punch (25) and received within the cavity (26) of the holding-down punch (25), wherein the holding-down punch (25) is prestressed, by means of a spring element (33), in the axial direction towards the patch receiving surface (22) of the holding-down punch (25), so that it projects above the setting punch (21) in the rest state, and a receiving space (30) for the repair patch (12) is formed.

10. A method for repairing a defect (5) in a wooden workpiece (1) by using a repair device (6), in particular a repair device (6) according to one of the preceding claims, comprising the method steps of:
- feeding a repair patch (12) to a setting punch (21);
- rotating the repair patch about a rotational axis (40) while simultaneously detecting, by means of a detection means (47), a surface of the repair patch (12), said surface having a marking (19), wherein the marking (19) indicates the grain direction (14) of the repair patch (12),
**characterized in that**
a first sensor (48) and a second sensor (49) of the detection means (47) detect the surface of the repair patch (12) at two detection regions (50, 51) arranged at a distance from one another, wherein the grain direction (14) of the repair patch (12) may be determined when both sensors (48, 49) detect the marking (19) arranged on the repair patch (12).

11. The method according to claim 10, **characterized in that** the sensors (48, 49) of the detection means (47) are arranged at the repair device (6) in such a way that after the marking (19) has been detected, the repair patch (12) is further rotated by a predetermined angle range until the final position of the repair patch (12) has been reached.

12. The method according to claim 10 or 11, **characterized in that** the marking (19) is arranged on a bottom side (18) of the repair patch (12).

13. The method according to one of claims 10 to 12, characterized that the marking (19) is arranged on the repair patch (12) in parallel to the grain direction (14).

14. The method according to one of claims 10 to 13, **characterized in that** the marking (19) is applied on the repair patch (12) in the form of a line running across the bottom side (18) of the repair patch (12), in particular by means of an ink-jet printer.

15. The method according to claim 14, **characterized in that** the marking (19) is applied to the repair patch (12) before the latter is cut out from a blank patch.

16. The method according to claim 15, **characterized in that** the detection regions (50, 51) of the sensors (48, 49) are arranged so as to be diametrically opposed with respect to the rotational axis (40).

17. The method according to one of claims 10 to 16, **characterized in that** the setting punch (21) is displaced relative to a machine frame for being positioned above the defect (5) in a wooden workpiece (1).

18. The method according to one of claims 10 to 17, **characterized in that** the wooden workpiece (1) is displaced relative to a machine frame.

## Revendications

1. Dispositif de réparation (6) pour la réparation d'un défaut (5) dans une pièce en bois (1), comprenant :
- un poinçon de pose (21) pour l'insertion d'un patch de réparation (12) dans un évidement (8) réalisé dans une pièce en bois (1) ;
- un dispositif d'alimentation (23) pour l'avance des patchs de réparation (12) vers le poinçon de pose (21) ;
- un moyen de détection (47) pour la détection de l'orientation du patch de réparation (12) à l'aide d'un marquage (19) disposé sur le patch de réparation (12) ;
dans lequel un dispositif rotatif (39) est prévu, qui permet de faire tourner le patch de réparation (12) autour d'un axe de rotation (40), **caractérisé en ce que** le moyen de détection (47) comprend un premier capteur (48) et un deuxième capteur (49), qui sont disposés sur le dispositif de réparation (6) de façon à permettre la détection du marquage (19) disposé sur le patch de réparation (12) sur des zones de détection (50, 51) distantes entre elles.

2. Dispositif de réparation selon la revendication 1, **caractérisé en ce que** le dispositif rotatif (39) comprend un poinçon de pose rotatif (21) qui peut tourner conjointement avec le patch de réparation (12).

3. Dispositif de réparation selon la revendication 2, **caractérisé en ce que** le dispositif rotatif (39) comprend une roue dentée (42), disposée sur le poinçon de pose rotatif (21), qui est couplée à l'aide d'un moyen de traction (43) avec un pignon (44) disposé sur un entraînement rotatif (45).

4. Dispositif de réparation selon la revendication 3, **caractérisé en ce que** l'entraînement rotatif (45) se présente sous la forme d'un servomoteur.

5. Dispositif de réparation selon l'une des revendications précédentes, **caractérisé en ce que** le poinçon de pose (21) comprend une surface de logement de patch (22) qui peut être sollicitée avec une dépression.

6. Dispositif de réparation selon l'une des revendications précédentes, **caractérisé en ce que** le poinçon de pose (21) peut être déplacé au moyen d'un actionneur d'actionnement (37), plus particulièrement un vérin pneumatique, dans la direction axiale de l'axe de rotation (40).

7. Dispositif de réparation selon l'une des revendications précédentes, **caractérisé en ce que** le moyen de détection (47) est positionné par rapport au poinçon de pose (21) de façon à ce qu'un côté inférieur (18) du patch de réparation (12) puisse être détecté.

8. Dispositif de réparation selon l'une des revendications précédentes, **caractérisé en ce que** le premier capteur (48) et le deuxième capteur (49) sont conçus comme un moyen de détection optique, plus particulièrement un capteur laser.

9. Dispositif de réparation selon l'une des revendications précédentes, **caractérisé en ce qu'**un poinçon de maintien cylindre creux (25) est prévu, dans lequel le poinçon de pose (21) est orienté de manière coaxiale par rapport au poinçon de maintien (25) et est logé à l'intérieur de la cavité (26) du poinçon de maintien (25), dans lequel le poinçon de maintien (25) est précontraint au moyen d'un élément de ressort (33) dans la direction axiale en direction de la surface de logement de patch (22) du poinçon de maintien (25), de façon à dépasser, à l'état de repos, du poinçon de pose (21) et un espace de logement (30) pour le patch de réparation (12) est prévu.

10. Procédé de réparation d'un défaut (5) dans une pièce en bois (1) à l'aide d'un dispositif de réparation (6), plus particulièrement d'un dispositif de réparation (6) selon l'une des revendications précédentes, comprenant les étapes suivantes :
- avance d'un patch de réparation (12) vers un poinçon de pose (21) ;
- rotation du patch de réparation autour d'un axe de rotation (40) et détection simultanée d'une surface du patch de réparation (12) munie d'un marquage (19), à l'aide d'un moyen de détection (47), dans lequel le marquage (19) indique la direction des fibres (14) du patch de réparation (12),
**caractérisé en ce que**
un premier capteur (48) et un deuxième capteur (49) du moyen de détection (47) détectent la surface du patch de réparation (12) sur deux zones de détection (50, 51) distantes entre elles, dans lequel la direction des fibres (14) du patch de réparation (12) peut alors être déterminée lorsque les deux capteurs (48, 49) détectent le marquage (19) disposé sur le patch de réparation (12).

11. Procédé selon la revendication 10, **caractérisé en ce que** les capteurs (48, 49) du moyen de détection (47) sont disposés sur le dispositif de réparation (6) de façon à ce que le patch de réparation (12), après la détection du marquage (19), soit encore tourné d'une plage angulaire prédéterminée jusqu'à ce que la position finale du patch de réparation (12) soit atteinte.

12. Procédé selon la revendication 10 ou 11, **caractérisé en ce que** le marquage (19) est disposé sur un côté inférieur (18) du patch de réparation (12).

13. Procédé selon l'une de revendications 10 à 12, **caractérisé en ce que** le marquage (19) est disposé parallèlement à la direction des fibres (14) sur le patch de réparation (12).

14. Procédé selon l'une de revendications 10 à 13, **caractérisé en ce que** le marquage (19) se présente sous la forme d'un trait s'étendant transversalement au-dessus du côté inférieur (18) du patch de réparation (12), plus particulièrement est appliqué, au moyen d'une imprimante à jet d'encre, sur le patch de réparation (12).

15. Procédé selon la revendication 14, **caractérisé en ce que** le marquage (19) est appliqué sur le patch de réparation (12) avant que celui-ci ne soit découpé à partir d'une ébauche de patch.

16. Procédé selon la revendication 15, **caractérisé en ce que** les zones de détection (50, 51) des capteurs (48, 49) sont disposées de manière diamétralement opposée par rapport à l'axe de rotation (40).

17. Procédé selon l'une de revendications 10 à 16, **caractérisé en ce que** le poinçon de pose (21) est déplacé par rapport à un cadre de machine, afin de placer celui-ci au-dessus du défaut (5) dans une pièce en bois (1).

18. Procédé selon l'une de revendications 10 à 17, **caractérisé en ce que** la pièce en bois (1) est déplacée par rapport à un cadre de machine.
